(19) Europäisches Patentamt
European Patent Office
Office européen des brevets

(11) EP 1 635 895 B1

(12) **FASCICULE DE BREVET EUROPEEN**

(45) Date de publication et mention
de la délivrance du brevet:
**24.10.2012 Bulletin 2012/43**

(21) Numéro de dépôt: **04743770.2**

(22) Date de dépôt: **17.06.2004**

(51) Int Cl.:
***A61M 1/16*** (2006.01)

(86) Numéro de dépôt international:
**PCT/IB2004/002024**

(87) Numéro de publication internationale:
**WO 2004/112869 (29.12.2004 Gazette 2004/53)**

(54) **DISPOSITIF DE TRAITEMENT DE SANG PAR CIRCULATION EXTRACORPORELLE A VIDANGE AUTOMATIQUE DE LIQUIDE USE**

EXTRAKORPORELLE BLUTBEHANDLUNGSVORRICHTUNG MIT AUTOMATISCHER ENTLEERUNG DER GEBRAUCHTEN FLÜSSIGKEIT

BLOOD PROCESSING DEVICE USING EXTRA-CORPOREAL CIRCULATION AND AUTOMATIC SPENT FLUID DRAINAGE

(84) Etats contractants désignés:
**AT BE BG CH CY CZ DE DK EE ES FI FR GB GR HU IE IT LI LU MC NL PL PT RO SE SI SK TR**

(30) Priorité: **25.06.2003 FR 0307643**

(43) Date de publication de la demande:
**22.03.2006 Bulletin 2006/12**

(60) Demande divisionnaire:
**11156262.5 / 2 338 542**

(73) Titulaire: **Gambro Lundia AB**
**22 643 Lund (SE)**

(72) Inventeur: **CHEVALLET, Jacques**
**F-69360 Serezin du Rhone (FR)**

(74) Mandataire: **Ponzellini, Gianmarco et al**
**Gambro Legal and Intellectual Property Dept.**
**P.O. Box 98**
**41037 Mirandola (IT)**

(56) Documents cités:
**EP-A- 0 796 997      EP-A1- 0 213 050**
**JP-A- 53 083 397      JP-A- 59 037 959**
**US-A- 4 267 041      US-A- 4 859 319**
**US-A- 5 043 074**

**Description**

Domaine technique de l'invention

La présente invention concerne le traitement extracorporel du sang, et plus particulièrement, un appareil innovant et amélioré pour le traitement du sang dans lequel on peut effectuer une vidange du liquide usé de façon automatique.

Etat de la technique antérieure

Le traitement extracorporel du sang implique de prélever du sang d'un patient, de traiter le sang extérieurement au patient, de renvoyer le sang traité au patient. Le traitement extracorporel de sang est utilisé typiquement pour extraire des matières ou des molécules indésirables du sang du patient, et/ou pour ajouter des matières ou des molécules bénéfiques au sang. Le traitement extracorporel de sang est utilisé avec des patients incapables d'éliminer efficacement des matières de leur sang, par exemple dans le cas d'un patient qui souffre d'une défaillance temporaire ou permanente des reins. Ces patients et d'autres patients peuvent suivre un traitement extracorporel du sang pour ajouter ou éliminer des matières dans leur sang, pour maintenir un équilibre acide-base ou pour éliminer des fluides corporels excessifs, par exemple.

Le traitement extracorporel du sang est typiquement effectué en prélevant le sang du patient en un flux continu, en introduisant le sang dans une chambre primaire d'un filtre dans laquelle le sang passe à travers une membrane semi- perméable. La membrane semi-perméable laisse passer, de manière sélective, des matières indésirables contenues dans le sang à travers la membrane, de la chambre primaire vers la chambre secondaire, et laisse également passer, de manière sélective, des matières bénéfiques contenues dans le liquide passant dans la chambre secondaire à travers la membrane vers le sang passant dans la chambre primaire, en fonction du type de traitement.

Un certain nombre de traitement extracorporel de sang peuvent être effectués par une même machine. Dans un traitement par ultrafiltration (UF), les matières indésirables sont éliminées du sang par convection à travers la membrane dans la chambre secondaire.

Dans un traitement par hémofiltration (HF), le sang s'écoule à travers la membrane semi-perméable comme dans l'UF, et des matières bénéfiques sont ajoutées au sang, typiquement par introduction d'un fluide dans le sang, soit avant, soit après son passage à travers le filtre et avant qu'il ne soit renvoyé au patient.

Dans un traitement par hémodialyse (HD), un fluide secondaire contenant des matières bénéfiques est introduit dans la chambre secondaire du filtre. Les matières indésirables du sang traversent la membrane semi-perméable et pénètrent dans le fluide secondaire et les matières bénéfiques du fluide secondaire peuvent traverser la membrane et pénétrer dans le sang.

Dans un traitement par hémodiafiltration (HDF), le sang et le fluide secondaire échangent leurs matières comme dans l'HD, et en outre, des matières sont ajoutées au sang, typiquement en introduisant un fluide dans le sang traité avant qu'il ne soit renvoyé au patient comme dans l'HF, et les matières indésirables sont aussi éliminées du sang par convection.

Dans chaque traitement, le fluide secondaire passe à travers la chambre secondaire du filtre et reçoit les matières indésirables du sang par le biais de la membrane. Ce liquide est ensuite extrait du filtre : il est communément appelé liquide usé, et est emmené vers un égout ou vers un recueil destiné ensuite à être vidé dans un égout.

Comme il a été décrit précédemment, un patient peut souffrir d'une défaillance temporaire ou d'une défaillance permanente des reins.

Dans le cas d'une défaillance permanente des reins, le patient devra subir d es séances régulières, par exemple trois fois par semaine, de traitement extracorporel sanguin avec un débit d'extraction de sang relativement élevé, à savoir entre 200 et 500mL/min. Généralement, le personnel soignant peut installer le dispositif de trait ement avec une préparation en ligne de liquide pouvant comporter une stérilisation en ligne, et peut installer un égout en ligne.

Dans le domaine d'une défaillance temporaire des reins, le patient devra être traité en urgence et devra subir un traitement extracorporel sanguin continu et de longue durée avec un débit d'extraction de sang relativement faible, c'est-à-dire entre 100 et 200mL/min. L'extraction nette d'eau du patient est limitée car le patient traité en urgence est dans un état critique.

Dans ce cas de traitement en urgence, le personnel soignant doit agir rapidement et n'a donc pas le temps d'installer l'appareil de traitement avec une préparation en ligne de liquide. Il est en effet beaucoup plus rapide d'accrocher au dispositif un liquide de dialyse et/ou un liquide d'infusion déjà préparé et stocké dans un sac jetable stérile, et il est plus rapide d'accrocher un sac jetable vide pour recueillir le liquide usé.

Une machine adoptant cette solution de sacs stériles jetables est connue. Pendant la séance de traitement intensif de l'insuffisance rénale, cette machine de traitement extracorporel doit assurer et contrôler plusieurs débits:

- le débit de perfusion (« infusion » en anglais) (Dinf), dans le cas où une infusion de liquide avec des matières bénéfiques est prescrite au patient,
- le débit de liquide de dialyse (Ddial) entrant dans la chambre secondaire du filtre dans le cas d'un mode HF ou HDF,
- le débit représentant la perte de poids (weight loss) par le patient (Dwloss), c'est-à-dire la quantité de liquide extraite et perdue par le patient,
- le débit représentant le liquide usé (waste) sortant du filtre Dwaste.

Le système représenté par le patient et l'appareil de traitement sanguin est un système fermé. Ainsi on peut en déduire l'équation suivante :

$$Dwaste = Dinf + Ddial + Dwloss \quad (1)$$

Aussi, avant la séance de traitement, le médecin peut prescrire :

- le débit de perfusion Dinf pour contrôler la quantité de matières bénéfiques à perfuser au patient,
- le débit de dialyse Ddial pour contrôler le passage de matières à travers le filtre,
- le débit de perte de poids Dwloss par le patient pour éviter tout malaise potentiel du patient pendant la séance.

Par conséquent le débit de liquide usé est calculé selon l'équation (1).

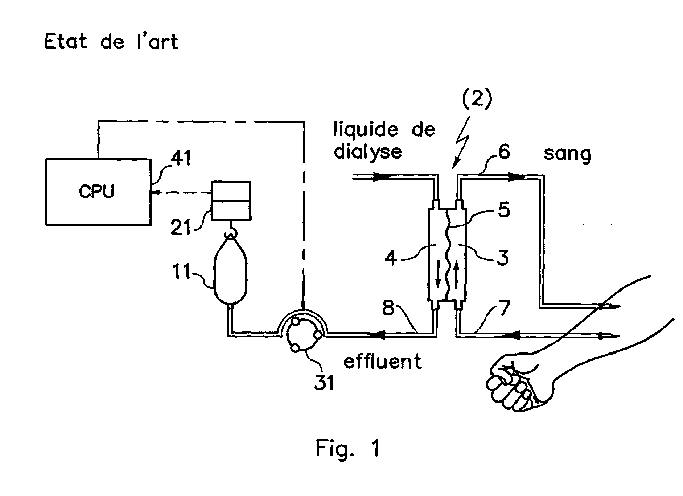

Pour cela, il a été décrit plus haut l'utilisation d'un sac jetable stérile permettant de recevoir et collecter le liquide usé. Cette utilisation connue est illustrée en figure 1. Le sac 11 est connecté à l'extrémité de la ligne 8 de liquide usé reliée à la chambre secondaire 4. Ce sac 11 est associé à un moyen de pesage gravimétrique 21 relié à une unité de contrôle 41. Ainsi, des signaux de poids sont transmis à l'unité de contrôle 41 qui est capable de suivre l'évolution du poids du sac liée au débit de liquide usé à travers la ligne 8 de liquide usé, et de commander une pompe 31 active sur la ligne de liquide usé.

Cependant, la séance peut durer plusieurs jours et le sac jetable de liquide usé se trouve rempli bien avant la fin de la séance. Ce phénomène est d'autant plus constaté lors d'un traitement intensif. En effet, on souhaite d'une part échanger une quantité de liquide élevée en thérapie HF ou HDF, et d'autre part on veut effectuer des traitements de longue durée.

Dès que le sac atteint un certain niveau de remplissage, le médecin ou l'infirmière intervient sur la machine pour arrêter temporairement les pompes respectivement actives sur la ligne de liquide usé, sur la ligne de liquide de dialyse et sur la ligne d'infusion, tandis que le sang continue à circuler extracorporellement dans la chambre primaire du filtre. Une fois les pompes arrêtées, l'utilisateur doit débrancher et décrocher le sac rempli de liquide usé, le vider et/ou le jeter dans un réseau de vidange. Puis l'utilisateur accroche et branche un nouveau sac vide à usage unique sur le dispositif de traitement et remet en fonctionnement les pompes pour revenir au traitement extracorporel avec circulation des fluides à travers les deux chambres (3, 4) du filtre 2.

Cette op ération de remplacement de sac présente des inconvénients :

- d'une part, elle peut durer quelques minutes et rallonge le temps de traitement de quelques minutes à chaque fois que le sac est rempli et nécessite d'être changé,
- d'autre part, cette opération de changement de sac s'effectue alors que le sang circule toujours dans le circuit sanguin sans pouvoir être en contact avec un liquide de dialyse passant, la qualité du traitement est dès lors moindre,
- aussi, cette opération est effectuée par du personnel soignant qui doit souvent suivre plusieurs patients en même temps. Un temps d'attente avant l'intervention du personnel peut encore s'ajouter au temps de traitement,
- en outre, le changement régulier du sac de vidange pendant une séance rajoute un coût économique au traitement,
- enfin, les sacs sont généralement d'un volume d'environ cinq litres, représentent un objet lourd et relativement fragile à manipuler et contiennent un liquide usé pouvant 5 représenter une source de substances indésirables si le sac était malencontreusement perforé pendant la manipulation.

Le document EP0796997 est relative à un dispositif de dialyse. Le dispositif comprends un premier et un second récipient reliés à un tube d'évacuation du liquide usé. Un première et un deuxième balance mesure le poids du liquide dans le premier et un second récipient.

Le document US 4859319 et le document US 50430745 concernent un dispositif de mesure de la quantité d'ultrafiltration éliminée pendant un traitement de dialyse. Le dispositif comprends un premier et un second récipient reliés à un tube d'évacuation et des sondes de mesure capable de détecter la présence d'ultrafiltrat à un niveau prédéterminé dans le premier et le second récipient et des moyens d'élaboration capables d'établir la quantité d'ultrafiltrat éliminé.

Exposé de l'invention:

La présente invention est décrite en référence particulière au traitement intensif de l'insuffisance rénale (encore appelé traitement de l'insuffisance rénale aiguë), sans par là limiter la portée de l'invention à cette application spécifique.

L'invention a pour objet un dispositif de traitement extracorporel sanguin ayant les mêmes fonctions que celui connu aujourd'hui et permettant de résoudre tous les problèmes décrits.

L'invention a pour objet un dispositif de traitement sanguin pour la vidange automatique et pour le contrôle du débit de liquide usé selon la revendication 1.

Brève description des dessins :

Des caractéristiques et avantages supplémentaires apparaîtront à la description détaillée d'un mode de réalisation préféré mais non exclusif d'un dispositif de traitement extracorporel sanguin selon l'invention. Cette description sera effectuée ci-dessous en référence aux dessins annexés, qui sont fournis à titre indicatif et donc non limitatif.

Il est décrit quatre modes de réalisation de l'invention.

La figure 1 représente un dispositif de traitement extracorporel de sang selon l'état connu de la technique.

La figure 2 représente un premier mode de réalisation du dispositif selon l'invention.

La figure 3 représente la première phase de fonctionnement du premier mode de réalisation du dispositif selon l'invention.

La figure 4 représente la seconde phase de fonctionnement du premier mode de réalisation du dispositif selon l'invention.

La figure 5 représente l'évolution du poids mesuré de sacs pendant la séance de traitement du premier mode de réalisation.

La figure 6 représente un deuxième mode de réalisation du dispositif selon l'invention.

La figure 7 représente la première phase de fonctionnement du deuxième mode de réalisation du dispositif selon l'invention.

La figure 8 représente la seconde phase de fonctionnement du deuxième mode de réalisation du dispositif selon l'invention.

La figure 9 représente un troisième mode de réalisation du dispositif selon l'invention.

La figure 10 représente la première phase de fonctionnement du troisième mode de réalisation du dispositif selon l'invention.

La figure 11 représente la seconde phase de fonctionnement du troisième mode de réalisation du dispositif selon l'invention.

La figure 12 représente un quatrième mode de réalisation du dispositif selon l'invention.

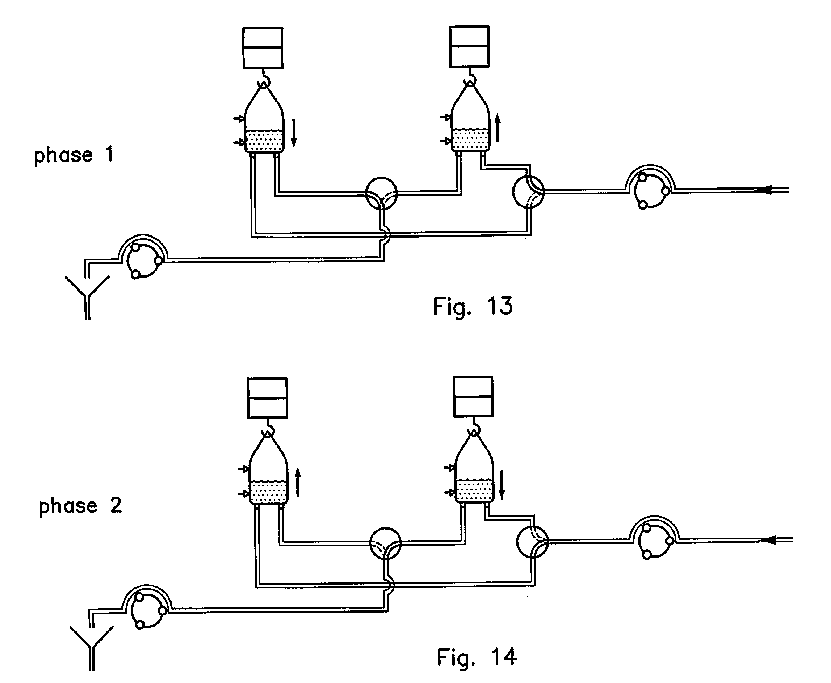

La figure 13 représente la première phase de fonctionnement du quatrième mode de réalisation du dispositif selon l'invention.

La figure 14 représente la seconde phase de fonctionnement du quatrième mode de réalisation du dispositif selon l'invention.

Exposé détaillé de modes de réalisation de l'invention

Description commune à tout mode :

En référence aux figures annexées, on a désigné globalement par 1 le dispositif de traitement extracorporel sanguin. Le dispositif de traitement de sang 1 est représenté dans les figures 1, 2, 6, 9 et 12 est dans une configuration fonctionnelle qui lui permet d'effectuer un traitement d'hémodialyse. Les autres configurations de traitement citées plus haut (ultrafiltration, hémofiltration et hémodiafiltration) sont bien entendu transposables aux modes de réalisation de l'invention.

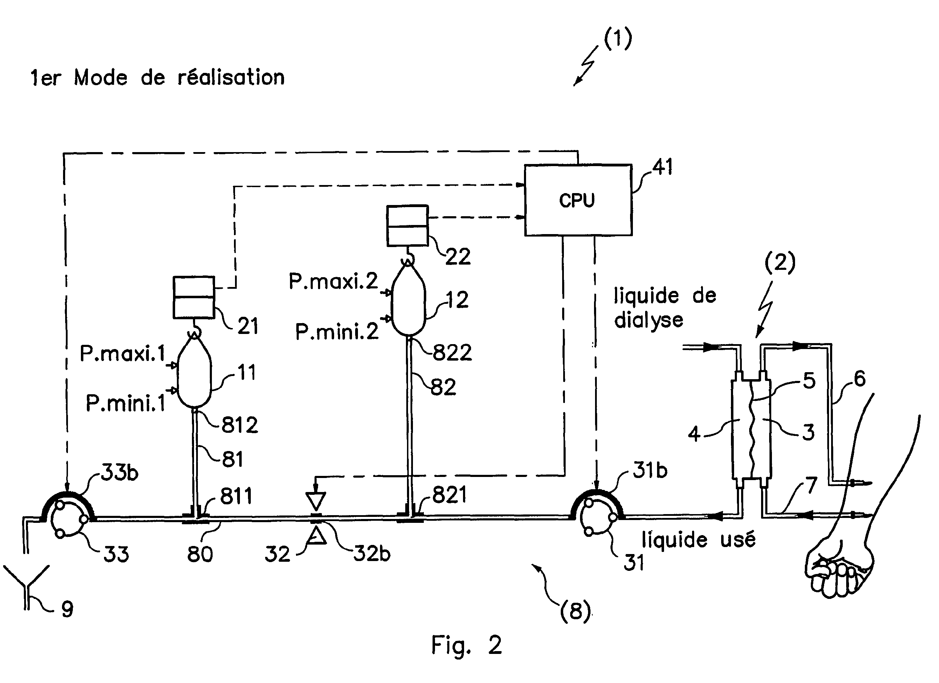

Les dispositifs selon les différents modes de réalisation de l'invention représenté dans les figures 2, 6, 9, 12 comportent un filtre 2 ayant une chambre primaire 3 et une chambre secondaire 4 séparées par une membrane semi-perméable 5 ; un circuit sang comporte une ligne artérielle 6 destinée à sortir du patient, la chambre primaire 3 du filtre et une ligne veineuse 7 destinée à retourner au patient ; un circuit dialysat comporte la chambre secondaire 4 du filtre et au moins une ligne de vidange 8 pour la circulation du liquide usé destiné à sortir du filtre 2 et destiné à aller vers un égout 9, un premier sac 11 en communication de fluide avec la ligne de vidange 8, au moins un premier moyen de pesage gravimétrique 21 associé au premier sac 11, des moyens de réglage de débit de fluide (31,32,33,34) actifs sur la ligne de vidange 8 ; une unité de contrôle 41 est reliée au premier moyen de pesage gravimétrique 21 et aux moyens de réglage de débit de fluide (31,32,33,34). Les dispositifs selon les différents modes de réalisation de l'invention comportent un second sac 12 en communication de fluide avec la ligne de vidange 8.

L'unité de contrôle 41 est capable de recevoir les signaux de poids du premier moyen de pesage gravimétrique et de commander les moyens de réglage de débit de fluide (31,32,33,34) pour charger de liquide un des sacs (11, 12) pendant que l'autre sac (12, 11) se décharge de liquide, et vice-versa.

Dans tout mode de réalisation, l'unité de contrôle 41 est également capable de calculer, à partir des signaux de poids reçus, la quantité de liquide sortant du filtre et entrant dans la ligne de vidange 8.

Plus particulièrement et dans tout mode de réalisation, les moyens de réglage comportent un premier organe de réglage 31 actif en amont des deux sacs (11, 12). Le premier organe est donc actif sur la ligne de vidange, après la sortie de la chambre secondaire du filtre, en amont des sacs. Aussi l'unité de contrôle 41 sera programmée pour commander le premier organe de réglage 31 afin de garantir la présence d'un débit substantiellement continu pendant le traitement. En effet, les moyens de réglage de débit sont commandés de sorte à ne plus devoir arrêter le flux de liquide usé sortant du filtre. Pour une qualité meilleure du traitement, on veillera à ce que le débit de liquide usé mesuré reste substantiellement constant ou suive un profil souhaité pendant la séance.

Dans tout mode de réalisation, les moyens de réglage peuvent comporter un deuxième organe de réglage 32 actif entre les deux sacs (11, 12).

Dans tout mode de réalisation, les moyens de réglage peuvent comporter un troisième organe de réglage 33 actif en aval des deux sacs (11, 12).

Des caractéristiques additionnelles sont possibles pour tout mode de réalisation.

Ainsi, le dispositif peut comporter un second moyen de pesage gravimétrique 22 associé au second sac 12 et relié à l'unité de contrôle 41. En effet, l'information de poids fournie à l'unité de contrôle par le premier moyen de pesage gravimétrique a pour fonction de connaître le poids du sac à usage unique à la fois pour savoir la quantité de liquide passant, et pour commander la phase de charge et décharge des deux sacs en utilisant deux valeurs seuils maximale et minimale prédéterminées par l'utilisateur en fonction de la contenance du sac.

L'association d'un second moyen de pesage gravimétrique au second sac est possible. Le second moyen de pesage gravimétrique a pour première fonction de connaître le poids du liquide passant. Il peut également servir au contrôle du processus cyclique de charge et décharge en utilisant une ou deux valeurs seuil du second moyen de pesage gravimétrique, en association avec une ou deux valeurs seuils du premier moyen de pesage gravimétrique. Mais si l'utilisation des deux valeurs seuils suffit pour le contrôle de la vidange, les quatre valeurs seuil des deux pesons peuvent être utilisées dans un but préventif d'alarme concernant l'état anormal d'un sac.

Dans tout mode de réalisation, l'unité de contrôle 41 est capable de calculer la quantité de fluide sortant du filtre 2 et entrant dans la ligne de vidange 8 à partir des signaux reçus du premier moyen de pesage gravimétrique 21 et/ou du second moyen de pesage gravimétrique 22.

Pour tout mode de réalisation, l'unité de contrôle 41 est capable d'activer une procédure de contrôle comportant deux phases alternées.

Dans une première phase, l'unité de contrôle commande le débit réel des moyens de réglage (31, 32, 33, 34) comme fonction du profil de débit souhaité et de l'information de poids venant d'au moins le premier moyen de pesage gravimétrique 21.

Dans une seconde phase, l'unité de contrôle 41 commande le débit réel des moyens de réglage (31, 32, 33, 34) comme fonction du profil de débit souhaité et de l'information de poids venant des premier et second moyens de pesage gravimétrique (21,22).

L'unité de contrôle 41 est également capable d'activer une procédure de contrôle comportant deux phases alternées avec un contrôle de débit réel des moyens de réglage (31, 32, 33, 34) lors de la première phase qui s'effectue également en fonction de l'information de poids du second moyen de pesage gravimétrique 22. Cette alternative peut être employée dans les troisième et quatrième modes de réalisation.

Dans tout mode de réalisation, l'unité de contrôle 41 est capable de recevoir l'information de poids du premier moyen de pesage gravimétrique 21 et/ou du second moyen de pesage gravimétrique 22, calculer le débit réel de fluide sortant du filtre 2 et le comparer à un débit souhaité prédéterminé ou à un profil de débit souhaité par l'utilisateur, contrôler le débit réel de fluide par les moyens de réglage pour approcher au meilleur le profil de débit souhaité de fluide sortant du filtre 2.

Dans tout mode de réalisation, l'unité de contrôle est capable de recevoir l'information de poids du premier moyen de pesage gravimétrique 21 et/ou du second moyen de pesage gravimétrique 22, déterminer indépendamment l'état de remplissage de chaque sac, commander, à partir de l'état de remplissage de chaque sac, une procédure de charge et décharge alternée et successive des sacs.

Dans tout mode de réalisation, l'unité de contrôle est capable de recevoir l'information de poids du premier moyen de pesage gravimétrique 21 et/ou du second moyen de pesage gravimétrique 22, détecter des valeurs de seuil maximal et seuil minimal pour chacun des sacs $P_{1mini}$, $P_{1maxi}$, $P_{2mni}$, $P_{2maxi}$, commander à partir de valeurs de seuil une procédure de charge et décharge des sacs selon les étapes suivantes :

o charge d'un sac et décharge de l'autre sac,
o détection d'un seuil limite,
o décharge d'un sac et charge de l'autre sac,
o détection d'un autre seuil limite.

Description commune au premier et deuxième modes de réalisation :

Dans les deux premiers modes de réalisation de l'invention, la ligne de vidange 8 peut être définie avec plusieurs composantes : un conduit 80 destiné à relier le filtre 2 à l'égout 9, un premier branchement 81 reliant le premier sac 11 au conduit 80, un deuxième branchement 82 reliant le second sac 12 au conduit 80. Le deuxième branchement 82 est connecté au conduit 80 en amont du premier branchement 81. Aussi le premier organe de réglage 31 et le deuxième organe de réglage 32 sont actifs sur le conduit 80, et non sur les deux branchements.

Chacun des deux branchements peut comporter une ligne avec deux raccords terminaux respectifs (811, 812, 821, 822), ou bien un raccord direct entre la ligne de vidange (80) et une ouverture d'un sac.

Description du premier mode de réalisation :

Dans le premier mode de réalisation illustré en figure 2, les premier 31 et troisième 33 organes de réglage sont opportunément des pompes péristaltiques, et le deuxième organe de réglage 32 est une vanne.

Dans le premier mode de réalisation illustré en figure 2, il faut tenir compte de la place dans l'espace des sacs. Il est connu que le ou les sacs jetables utilisés sont accrochés à la machine avec l'ouverture des sacs placée opportunément vers le bas pour permettre un écoulement du fluide continu. On peut trou ver plusieurs sacs notamment un sac jetable collectant le liquide usé, un sac jetable contenant le liquide de perfusion, un sac jetable comportant un liquide de dialyse. Ces sacs sont souvent accrochés au même niveau.

L'invention utilise la gravité pour faciliter l'écoulement de liquide sans forcément recourir à une pompe supplémentaire. Cette utilisation est faite dans le premier mode de réalisation, mais la gravité pourrait être utilisée dans les autres modes par l'homme du métier à l'aide de ses connaissances et l'exposé de l'invention.

Ainsi, le premier sac 11 est placé plus bas que le second sac 12 sur la machine. Par conséquent, lorsqu'un liquide usé est amené depuis la chambre secondaire du filtre et qu'un passage de fluide est possible entre les d eux sacs, le premier sac 11 se chargera en priorité par rapport au second sac, même si le second sac est placé en amont du premier sac dans le sens de circulation du fluide. De la même façon, lorsque le second sac sera rempli et le deuxième substantiellement vide, le second sac 12 se déchargera dans le premier sac 11 par gravité.

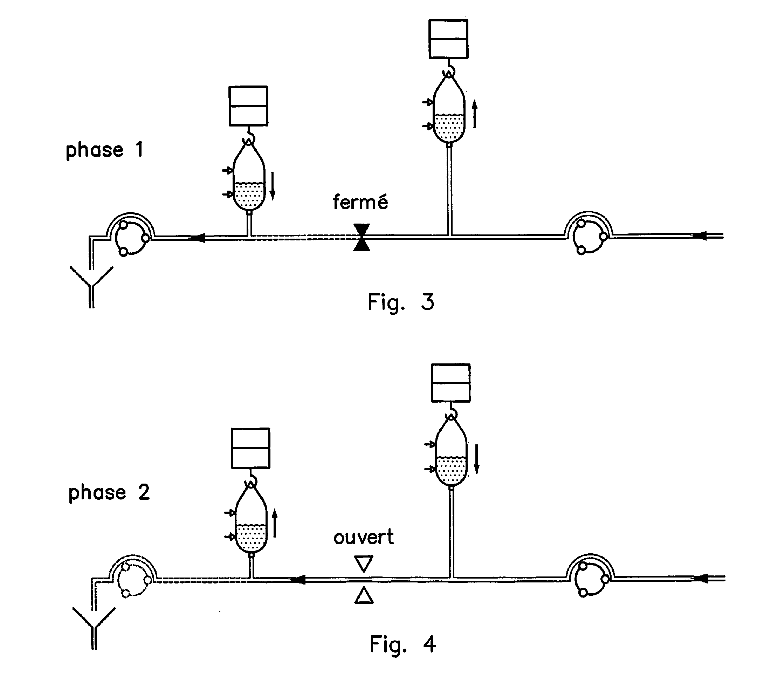

Les figures 3 et 4 illustrent les deux phases de fonctionnement du cycle de vidange instauré par l'appareil et les sens de passage de fluide dans la ligne de vidange, pour le premier mode de réalisation.

En effet, l'unité de contrôle 41 est capable de commander les organes de réglage (31, 32, 33) selon deux phases alternées.

En première, phase l'unité de contrôle 41 commande La fermeture du deuxième organe de réglage 32 pour la charge du second sac 12 et la décharge du premier sac 11 dans l'égout 9. En seconde phase, l'unité de contrôle 41 commande l'ouverture du deuxième organe de réglage 32 et l'arrêt du troisième organe de réglage 33 pour la décharge du second sac 12 et la charge du premier sac 11.

On notera que la commande pour passer d'une phase à l'autre doit s'effectuer de manière opportune de façon simultanée afin d'avoir une meilleure qualité de traitement, mais un petit intervalle de temps pourra être constaté entre deux actionnements, par exemple entre l'ouverture du deuxième organe de réglage 32 et l'arrêt du troisième organe de réglage 33. Ceci est valable pour toute commande de tout moyen de réglage.

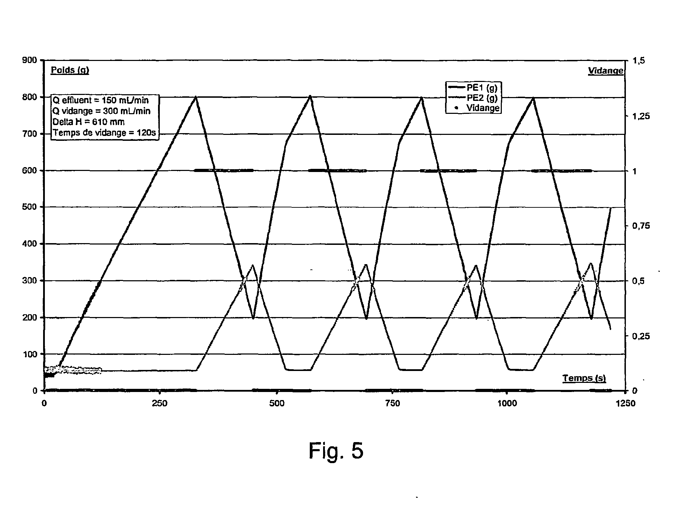

La figure 5 représente, pour le premier mode de réalisation, l'évolution du poids de chaque sac en fonction du temps de traitement. Ces mesures ont été effectuées expérimentalement et sont reproductibles.

On va maintenant expliquer, à partir du cas particulier de la figure 5, la succession des deux phases pendant le cycle de vidange, précédée d'une phase d'amorçage du système.

Au début de la séance, les deux sacs sont presque vides (un poids de 50g est enregistré), une phase d'amorçage est mise en oeuvre.

L'unité de contrôle amorce le premier moyen de réglage 31, ouvre le deuxième organe de réglage 32, et ne fait pas fonctionner le troisième moyen de réglage 33.

Le premier organe de réglage commande le passage du liquide usé dans la ligne de vidange 8. Dès lors, le liquide va passer dans le conduit 80.

Or le premier sac 11 est en aval par rapport au second sac 12, mais est accroché au dispositif plus bas que le second sac. Plus particulièrement, la limite haute du premier sac est placée plus bas ou au même niveau que la limite basse du second sac. Le second sac se charge en priorité par rapport au premier sac.

Ainsi, on constate que le poids du premier sac 11 augmente régulièrement en priorité par rapport au poids du second sac 12 qui reste inchangé.

Dès que le premier sac 11 atteint un poids maximal prédéterminé $P_{1maxi}$ (800g pour l'essai), le dispositif va fonctionner selon une première phase: le premier organe de réglage 31 continue à fonctionner, le second organe de réglage 32 est fermé et le troisième organe de réglage fonctionne pour conduire le liquide vers l'égout.

Dès lors le premier sac 11 dont le poids aura été mémorisé par l'unité de contrôle, va se décharger dans l'égout. On constate que le poids du premier sac diminue régulièrement de 800g à 200g.

D'autre part, le second sac se charge avec le liquide usé sortant du filtre. On constate une augmentation du poids du second sac 22 de 50g à 330g environ.

Cette phase s'effectuera jusqu'à ce que, soit un seuil minimal de poids du premier sac $P_{1mini}$ est atteint (200g), soit un seuil maximal de poids du second sac $P_{2maxi}$ (330g) est atteint, soit le premier des deux seuils précités est atteint.

A la détection d'un tel seuil, l'unité de contrôle commande l'entrée en seconde phase.

L'unité 41 commande l'ouverture du deuxième organe de réglage 32 et l'arrêt du fonctionnement du troisième organe de réglage 33. Ainsi, le second sac presque rempli, dont l'information de poids pourra être mémorisée par l'unité de contrôle, se décharge dans le premier sac presque vide. On constate que le premier sac se remplit non seulement avec le liquide contenu dans le second sac 22 mais aussi avec le liquide sortant directement du filtre. C'est pourquoi on constate une inflexion de la droite représentant l'augmentation régulière de poids pendant la seconde phase : le second sac est presque vidé à cet instant et la charge du premier sac s'effectuera moins rapidement (50g).

Ainsi vont s'alterner première et seconde phase jusqu'à la fin de la séance.

La taille de chacun des sacs, la taille des lignes jetables sont prédéterminées par l'utilisateur avant la séance. Dans l'essai conduit avec le premier mode de réalisation, le second sac a une contenance de 500g environ alors que le premier sac a une plus grande contenance, d'environ 1 kg, les lignes ont la même taille. Les débits adoptés pendant la séance s'accordent bien entendu à la taille des sacs et ligne et sont tels que le premier sac 11 atteint un poids minimal prédéterminé avant que le second sac 12 n'atteigne un poids maximal prédéterminé. Dans l'essai illustré, le débit de vidange est de 300mL/min et le débit au travers du premier organe réglage est de 150mL/min.

Description du deuxième mode de réalisation:

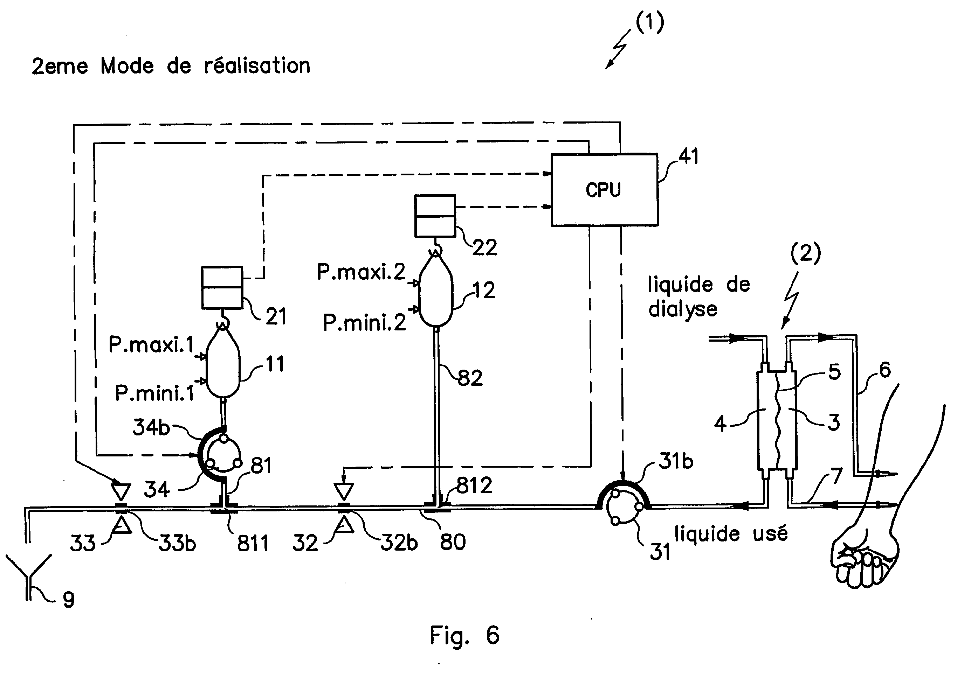

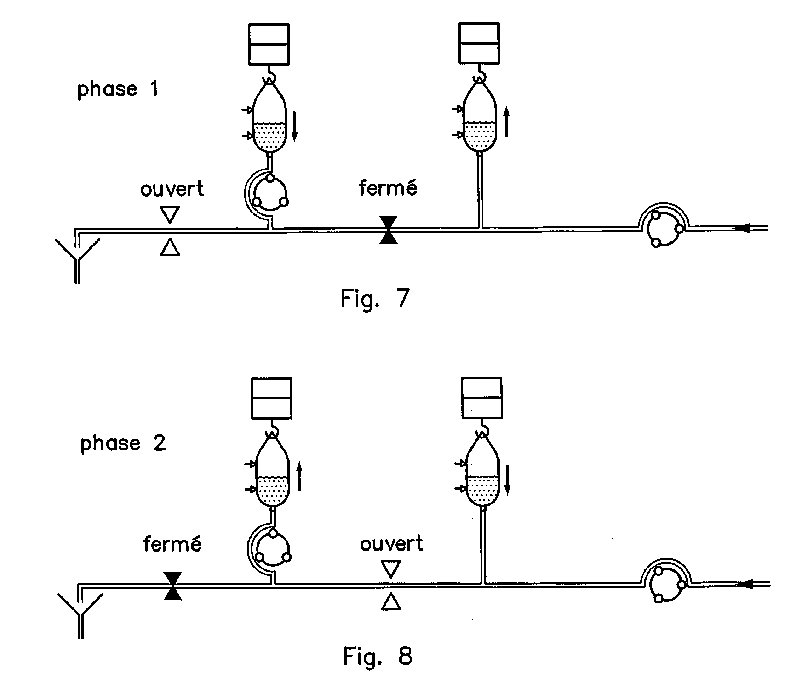

Un deuxième mode de réalisation est illustré en figure 6 et les deux phases de fonctionnement sont représentées en figure 7 et 8.

Dans le deuxième mode de réalisation, les moyens de réglage comportent un quatrième organe de réglage 34 actif sur le premier branchement B1 entre le raccord (811) et le premier sac (11).

Ce quatrième organe de réglage 34 peut comporter indifféremment une pompe, plus particulièrement une pompe péristaltique, une vanne, plus particulièrement un clamp deux voies ou une vanne à ouverture réglable.

Plus particulièrement dans le deuxième mode de réalisation, le premier et le quatrième organes de réglage (31, 34) peuvent être des pompes péristaltiques et les deuxième et troisième organes de réglage (32,33) peuvent être des vannes.

Dans le deuxième mode de réalisation, l'unité de contrôle 41 est capable de commander les moyens de réglage de débit (31, 32, 33, 34) selon deux phases alternées.

En première phase, l'unité de contrôle 41 commande la fermeture du deuxième organe de réglage 32, l'ouverture du troisième organe de réglage 33, l'actionnement du quatrième organe de réglage 34 dans le sens sac-conduit,

En seconde phase, l'unité de contrôle 41 commande l'ouverture du deuxième organe de réglage 32, la fermeture du troisième organe de réglage 33, l'actionnement du quatrième organe de réglage 34 dans le sens conduit-sac.

Description commune aux troisième et quatrième modes de réalisation :

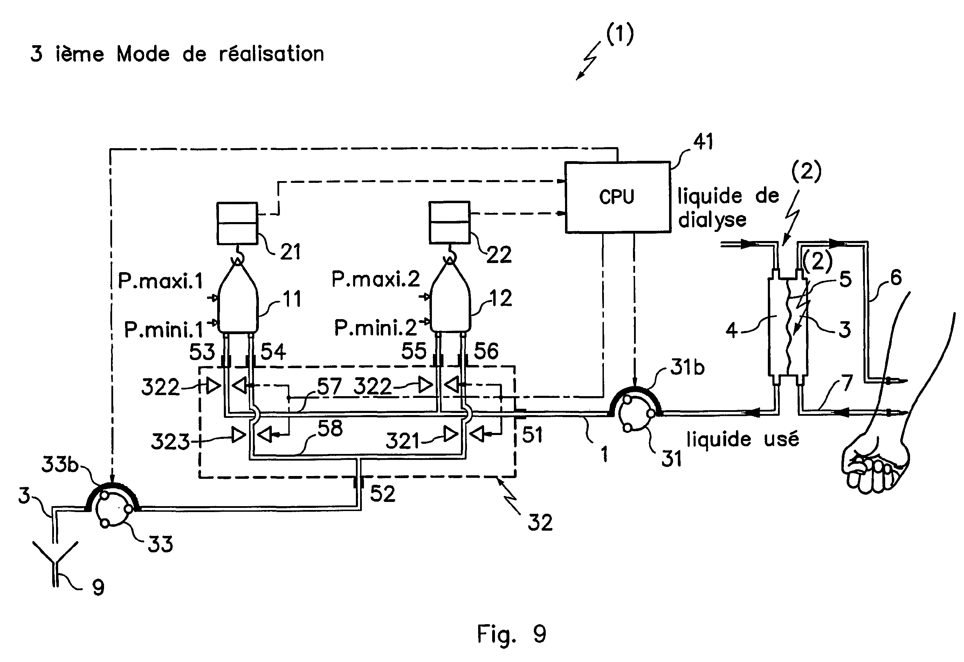

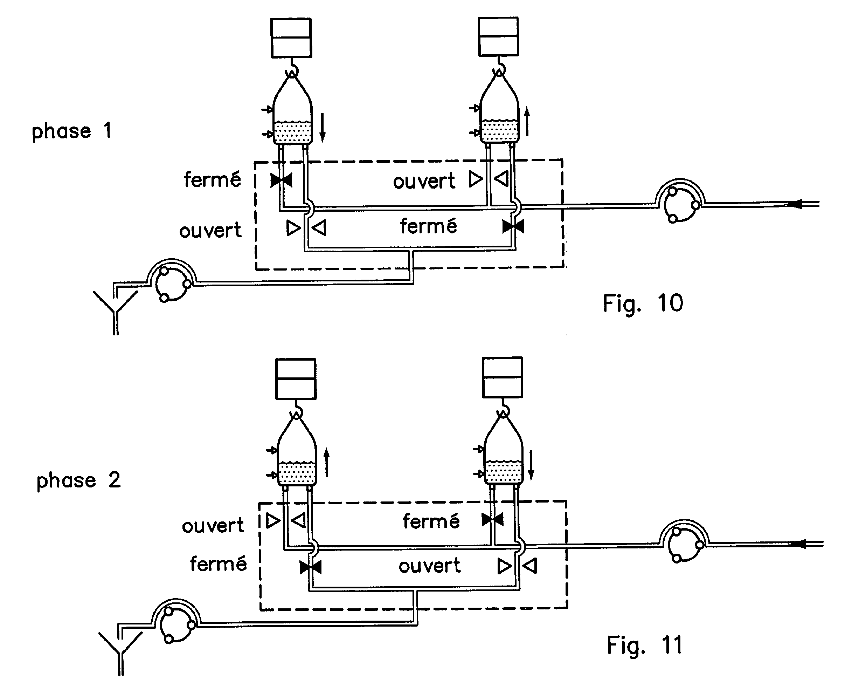

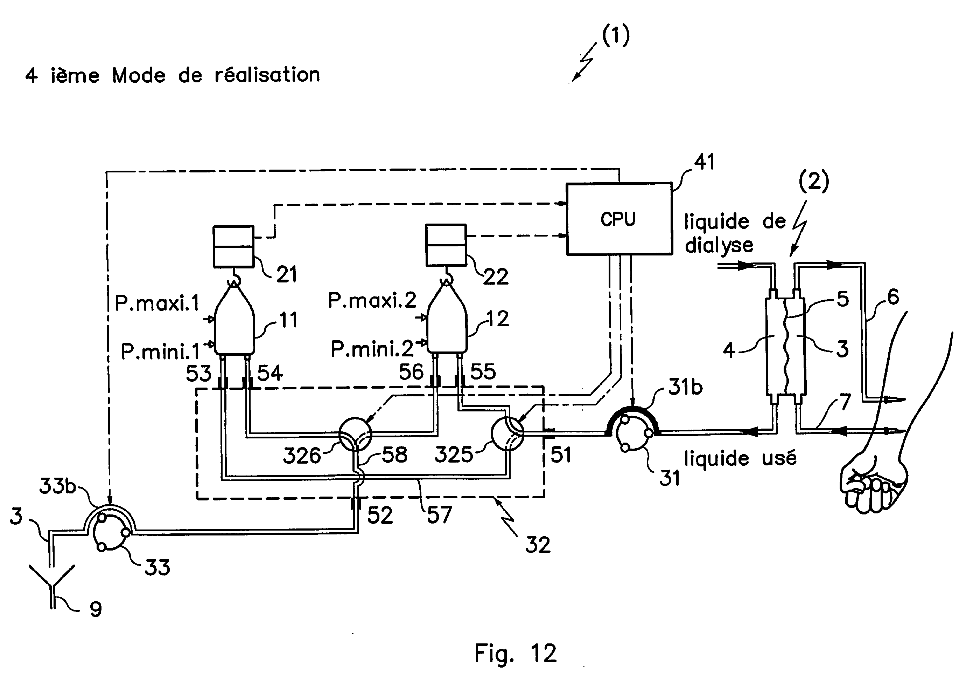

L'invention comporte également un troisième et un quatrième modes de réalisation illustrés respectivement dans les figures 9 et 12 et dont les deux phases de fonctionnement sont représentées respectivement dans les figures 10 et 11 ainsi que 13 et 14.

Dans ces deux modes, le deuxième organe de réglage 32 comporte un circuit hydraulique ayant six accès (51, 52, 53, 54, 55, 56) répartis de la façon suivante:

    a) un premier accès en entrée 51 destiné à être en communication de fluide avec la portion d'entrée de la ligne de vidange 8 destinée à être reliée au filtre,

    b) un deuxième accès en sortie 52 destiné à être en communication de fluide avec la portion de sortie de la ligne de vidange 8 destinée à être reliée à l'égout,

    c) un troisième accès en entrée 53 et un quatrième accès en sortie 54 destinés chacun à être en communication de fluide avec le premier sac 11,

    d) un cinquième accès en entrée 55 et un sixième accès en sortie 56 destinés chacun à être en communication de fluide avec le deuxième sac 12.

Description du troisième mode de réalisation :

Dans le troisième mode de réalisation, représenté en figure 9, le circuit hydraulique du deuxième organe de réglage 32 comporte deux parties.
La première partie comporte une première ligne 57 destinée à mettre en communication de fluide le premier accès en entrée 51 avec chacun des deux accès en sortie (54 et 56) destinés à communiquer avec chaque sac et deux clamps (322, 324) placés respectivement sur chaque portion de la première ligne 57 connectée auxdits deux accès en sortie (54 et 56). La seconde partie comporte une deuxième ligne 58 destinée à mettre en communication de fluide le deuxième accès en sortie 52 avec chacun des deux accès en entrée (53 et 55) destinés à communiquer avec chaque sac, deux autres clamps (321, 323) placés respectivement sur chaque portion de la deuxième ligne 58 connectée auxdits deux accès en entrée (53 et 55).

Description du quatrième mode de réalisation :

Dans le quatrième mode de réalisation, représenté en figure 12, la structure du circuit hydraulique du deuxième organe de réglage 32 est différente, même si le fonctionnement sera le même.
En effet, le deuxième moyen de réglage 32 comporte deux parties.
La première partie comporte une première ligne 57 destinée à mettre en communication de fluide le premier accès en entrée 51 avec chacun des deux accès en sortie (53, 55) destinés à communiquer avec chaque sac et un premier clamp trois voies 325 pouvant adopter deux positions alternées. La première position est la mise en communication de fluide du premier accès en entrée 51 avec le troisième accès en sortie 53 au niveau du premier sac 11. La seconde position est la mise en communication de fluide du premier accès en entrée 51 avec le cinquième accès en sortie 55 au niveau du deuxième sac 12.
La seconde partie comporte une deuxième ligne 58 destinée à mettre en communication de fluide le deuxième accès en sortie 52 avec chacu n des deux accès en entrée (54, 56) destinés à communiquer avec chaque sac et un deuxième clamp trois voies 326 pouvant adopter deux positions alternées suivantes. Une première position est la mise en communication de fluide du deuxième accès en sortie 52 avec le sixième accès en entrée 56, au niveau du deuxième sac 12. La seconde position est la mise en communication de fluide du deuxième accès en sortie 52 avec le quatrième accès en entrée 54, au niveau du premier sac 11.
Et, pour le troisième ou le quatrième mode de réalisation, le mode de fonctionnement alterné de charge et de décharge est identique. En effet, l'unité de contrôle 41 commande simultanément les clamps (321, 323, 323, 324) du deuxième organe de réglage 32 de sorte que deux phases sont alternées durant le fonctionnement.
En première phase, le deuxième sac 12 se charge de liquide tandis que le premier sac 11 se décharge vers l'égout 9.
En deuxième phase, le premier sac 11 se charge de liquide usé tandis que le deuxième sac 12 se décharge vers l'égout 9.
Il faut remarquer que pendant les deux phases, le troisième organe de réglage (33) assure un débit substantiellement continu, c'est-à-dire que du liquide usé est continuellement envoyée vers l'égout.
L'invention concerne également une ligne jetable ou à usage unique (« disposable » en anglais) pour l'utilisation dans le dispositif selon l'invention.
Dans tout mode de réalisation, cette ligne jetable comprend au moins deux sacs et quatre parties de lignes dont :

-   une première partie de ligne destinée à conduire du liquide depuis l'entrée de la ligne jetable (80) vers un des deux sacs (11,12);
-   une deuxième partie de ligne destinée à conduire du liquide contenu dans ledit sac vers la sortie de la ligne jetable (80);
-   une troisième partie de ligne destinée à conduire du liquide depuis l'entrée de la ligne jetable (80) vers l'autre sac (12, 11);
-   une quatrième partie de ligne destinée à conduire du liquide stocké dans l'autre sac (12, 11) vers la sortie de la ligne jetable (80).

Pour les premier et deuxième mode de réalisation décrits, les première et troisième parties ont un tronçon commun reliant les deux sacs.
En effet, dans le premier et le deuxième mode de réalisation : la première partie de ligne est constituée par une partie du conduit 80 depuis l'entrée de la ligne 80 jusqu'au deuxième raccord 82 ou 821 et par le deuxième tronçon ou raccord 82. La deuxième partie de la ligne est constituée par le tronçon ou raccord 82 et par une partie du conduit 80 entre le racco rd 821 ou 82 et la sortie de la ligne vers l'égout. La troisième partie de la ligne est constituée par une partie du conduit 80 depuis l'entrée de la ligne jusqu'au premier raccord 81 ou 811 et par le premier branchement ou raccord 81. La quatrième partie de la ligne est constituée par le premier raccord ou branchement 82 et par la partie du conduit 80 entre le premier raccord ou branchement 81 ou 811 et la sortie de la ligne.

Dans tout mode de réalisation, la ligne jetable comprend une ligne de vidange 80 destinée raccorder la sortie du filtre 2 à l'égout 9, deux sacs (11, 12) rattachés chacun à la ligne de vidange 8 et destinés à être accrochés à l'appareil de traitement 1, et au moins deux parties (31b, 33b) de la ligne de vidange 8 destinées à coopérer respectivement avec le premier organe de réglage 31 et le troisième organe de réglage 33.

Dans les premier et deuxième modes de réalisation, la ligne jetable comprend un conduit 80 , et au moins deux raccordements (81, 82) sur le conduit 80.

Dans le pr emier mode de réalisation, la ligne jetable comporte une autre partie 32b du conduit 80 placée entre les deux raccordements et destinée à coopérer avec le deuxième organe de réglage 32.

Dans le deuxième mode de réalisation, la ligne jetable comprend une quatrième partie (34b) placée sur le premier branchement (81) et destinée à coopérer avec le quatrième organe de réglage (34).

Dans le troisième mode de réalisation, la ligne jetable comprend une portion d'entrée de la ligne, une ligne de vidange 8 et une portion de sortie de la ligne.

La ligne de vidange 8 comporte une première canalisation de ligne 57 destiné à mettre en communication de fluide la portion d'entrée de la ligne de vidange et un accès à chaque sac, la première ligne étant en forme de T, et comporte une deuxième canalisation de ligne 58 destiné à mettre en communication de fluide la portion de sortie de la ligne de vidange et un second accès à chaque sac, la deuxième ligne étant en forme de T.

Dans le quatrième mode de réalisation, la ligne jetable comprend une portion d'entrée de la ligne, une ligne de vidange 8 et une portion de sortie de la ligne.

La ligne de vidange 8 comporte une première canalisation 57 destiné à mettre en communication de fluide la portion d'entrée de la ligne de vidange et un accès à chaque sac, la première canalisation comportant une vanne trois voies avec deux entrées et une sortie pour la connexion sélective de la sortie avec une des deux sorties,

La ligne de vidange 8 comporte également une deuxième canalisation 58 destiné à mettre en communication de fluide la portion de sortie de la ligne de vidange et un second accès à chaque sac, la deuxième canalisation comportant une vanne trois voies avec deux entrées et une sortie pour la connexion sélective de la sortie avec une des deux sorties.

Une telle ligne jetable peut être placée avant le début de la séance sur le dispositif de traitement extracorporel. A la fin de la séance, cette ligne sera déconnectée, jetée et remplacée par une nouvelle ligne pour la séance suivante.

L'invention concerne également un procédé de vidange automatique d'une ligne de vidange, correspondant au dispositif selon l'invention.

Le procédé comporte deux phases alternées successives ayant les étapes suivantes : le pas sage en continu d'un liquide usé à travers une ligne de vidange en sortie d'un filtre, la première phase et la deuxième phase successive et alternée à la première phase.

La première phase comporte la charge d'un premier conteneur (sac par exemple) par le liquide usé et la décharge d'un second conteneur (sac par exemple), l'atteinte d'un premier poids seuil mesuré. La seconde phase comporte la décharge du premier conteneur de liquidé vers un égout et charge du second conteneur de liquide usé, et l'atteinte d'un second poids seuil mesuré.

En d'autres termes, le procédé de vidange automatique peut comporter deux phases alternées successives:

- la première phase comporte la charge d'un premier sac (11, 12) par un liquide usé et la décharge d'un second sac (12,11) de liquide usé vers un égout en sortie de la ligne de vidange, cette phase s'arrêtant dès l'atteinte par au moins un des deux sacs (11, 12) d'un premier poids seuil mesuré,
- la seconde phase comporte la décharge du premier sac de liquide (11, 12) vers un égout en sortie de la ligne de vidange et la charge du second sac (12, 11) de liquide usé, cette phase s'arrêtant dès l'atteinte d'un second poids seuil mesuré sur le même sac et/ou sur l'autre sac.

Avantages de l'invention :

Les multiples avantages obtenus par l'invention sont les suivants :

- un contrôle du débit de liquide usé passant à travers la ligne de vidange est connu et contrôlé,
- la durée de séance avec le dispositif à vidange automatique est moindre à la durée d'une séance sans vidange automatique,
- le personnel soignant n'a plus à intervenir pour effectuer l'opération de changement de sac,
- le poids du liquide usé extrait du filtre est connu et maîtrisé
- les résultats, qualités du traitement utilisé selon l'état de l'art sont conservés,
- le niveau de sécurité assuré par le dispositif de traitement est conservé,

- l'équilibre hydrique est maintenu,
- le coût du traitement est diminué car deux sacs sont utilisés au lieu dé plusieurs sacs remplacés successivement,
- le premier sac associé à son premier moyen de pesage gravimétrique et la portion de sortie de la ligne de vidange et l'égout peuvent être placés non pas sur le dispositif, mais placé dans un dispositif ou une pièce séparée du dispositif de traitement afin d'assurer une parfaite séparation entre le dispositif et le patient et la vidange : ceci renforce la sécurité du traitement.

**Revendications**

1. Dispositif de traitement de sang par circulation extracorporelle (1) comportant :

   - un filtre (2) ayant une chambre primaire (3) et une chambre secondaire (4) séparées par une membrane semi-perméable (5),
   - un circuit sang comportant une ligne artérielle (6) destinée à sortir du patient, la chambre primaire (3) du filtre et une ligne veineuse (7) destiné à retourner au patient,
   - un circuit dialysat comportant la chambre secondaire (4) du filtre et une ligne de vidange (8) pour la circulation du liquide usé destiné à sortir du filtre (2) et destiné à aller vers un égout (9),
   - un premier sac (11) en communication de fluide avec la ligne de vidange (8),
   - au moins un premier moyen de pesage gravimétrique (21) associé au premier sac (11),
   - des moyens de réglage de débit de fluide (31,32, 33,34) actifs sur la ligne de vidange (8),
   - une unité de contrôle (41) reliée au premier moyen de pesage gravimétrique (21) et aux moyens de réglage de débit de fluide (31,32, 33,34),
   - un second sac (12) en communication de fluide avec la ligne de vidange (8),
   - un second moyen de pesage gravimétrique (22) associé au second sac (12) et relié à unité de contrôle (41),
   - ladite unité de contrôle (41) étant capable de:

      recevoir les signaux de poids du premier moyen de pesage gravimétrique et,
      commander les moyens de réglage de débit de fluide (31,32, 33,34) pour charger de liquide un des sacs (11,12) pendant que l'autre sac (12, 11) se décharge de liquide, et vice-versa afin d'obtenir une procédure de charge et décharge alternée et successive des sacs, et ladite unité de contrôle (41) étant capable de:

         recevoir l'information de poids du premier moyen de pesage gravimétrique (21) et du second moyen de pesage gravimétrique (22),
         calculer le débit réel de fluide sortant du filtre (2) et le comparer à un débit souhaité, et
         contrôler le débit réel de fluide par les moyens de réglage pour approcher le débit souhaité de fluide sortant du filtre (2).

2. Dispositif selon la revendication 1 **caractérisé en ce que** l'unité de contrôle (41) est capable de calculer, à partir des signaux de poids reçus, la quantité de liquide sortant du filtre et entrant dans la ligne de vidange (8).

3. Dispositif selon la revendication 1 ou 2 **caractérisé en ce que** les moyens de réglage comportent un premier organe de réglage (31) actif en amont des deux sacs (11, 12) et **en ce que** l'unité de contrôle (41) commande le premier organe de réglage (31) pour garantir la présence d'un débit substantiellement continu pendant le traitement.

4. Dispositif selon la revendication 3 **caractérisé en ce que** les moyens de réglage comportent un deuxième organe de réglage (32) actif entre les deux sacs (11, 12).

5. Dispositif selon la revendication 4 **caractérisé en ce que** les moyens de réglage comportent un troisième organe de réglage (33) actif en aval des deux sacs (11, 12).

6. Dispositif selon une des revendications 4 à 5, **caractérisé en ce que** la ligne de vidange (8) comprend :

   - un conduit (80) destiné à relier le filtre (2) à l'égout (9),
   - un premier branchement (81) reliant le premier sac (11) au conduit (80),
   - un deuxième branchement (82) reliant le second sac (12) au conduit (80),
   - le deuxième branchement (82) étant connecté au conduit (80) en amont du premier branchement (81),

et **en ce que** le premier organe de réglage (31) et le deuxième organe de réglage (32) sont actifs sur le conduit (80).

7. Dispositif selon la revendication 6 **caractérisé en ce que** chaque branchement (81, 82) comporte une ligne avec deux raccords terminaux respectifs (811, 812, 821, 822).

8. Dispositif selon la revendication 6 ou 7 **caractérisé en ce que** chaque branchement (81, 82) comporte un raccord direct entre la ligne de vidange (80) et une ouverture d'un sac.

9. Dispositif selon l'une des revendications 5 et 6 à 8 **caractérisé en ce que** les premier (31) et troisième (33) organes de réglage sont des pompes péristaltiques, et le deuxième organe de réglage (32) est une vanne.

10. Dispositif selon la revendication 9 **caractérisé en ce que** le premier sac (11) est placé plus bas que le second sac (12) de sorte que le premier sac (11) se charge en priorité et le second sac (12) se décharge dans le premier sac (11) par gravité lorsque le deuxième organe de réglage (32) est ouvert.

11. Dispositif selon la revendication 9 ou 10 **caractérisé en ce que** l'unité de contrôle (41) est capable de commander les organes de réglage (31, 32, 33) selon les deux phases alternées suivantes :

- en première phase : l'unité de contrôle (41) commande la fermeture du deuxième organe de réglage (32) pour la charge du second sac (12) et la décharge du premier sac (11) dans l'égout (9),
- en seconde phase : l'unité de contrôle (41) commande l'ouverture du deuxième organe de réglage (32) et l'arrêt du troisième organe de réglage (33) pour la décharge du second sac (12) et la charge du premier sac (11).

12. Dispositif selon la revendication 5 et 7 **caractérisé en ce que** les mo yens de réglage comportent un quatrième organe de réglage (34) actif sur le premier branchement (81) entre les raccord (811) et le premier sac (11).

13. Dispositif selon la revendication 12 **caractérisé en ce que** le premier et le quatrième organes de réglage (31, 34) sont des pompes péristaltiques et les deuxième et troisième organes de réglage (32,33) sont des vannes.

14. Dispositif selon la revendication 13 **caractérisé en ce que** l'unité de contrôle (41) est capable de commander les moyens de réglage de débit (31, 32, 33, 34) selon les deux phases alternées suivantes:

- en première phase : l'unité de contrôle (41) commande la fermeture du deuxième organe de réglage (32), l'ouverture du troisième organe de réglage (33), l'actionnement du quatrième organe de réglage (34) dans le sens sac-conduit,
- en seconde phase : l'unité de contrôle (41) commande l'ouverture du deuxième organe de réglage (32), la fermeture du troisième organe de réglage (33), l'actionnement du quatrième organe de réglage (34) dans le sens conduit-sac.

15. Dispositif selon une des revendications 4 à 5 **caractérisé en ce que** le deuxième organe de réglage (32) comporte un circuit hydraulique ayant six accès (51, 52, 53, 54, 55, 56) répartis de la façon suivante:

a. un premier accès en entrée (51) destiné à être en communication de fluide avec la portion d'entrée de la ligne de vidange (8) destinée à être reliée au filtre (2),
b. un deuxième accès en sortie (52) destiné à être en communication de fluide avec la portion de sortie de la ligne de vidange (8) destinée à être reliée à l'égout (9),
c. un troisième accès en entrée (53) et un quatrième accès en sortie (54) destinés chacun à être en communication de fluide avec le premier sac (11),
d. un cinquième accès en entrée (55) et un sixième accès en sortie (56) destinés chacun à être en communication de fluide avec le deuxième sac (12).

16. Dispositif selon la revendication 15 **caractérisé en ce que** le circuit hydraulique du deuxième organe de réglage (32) comporte:

- une première ligne (57) destinée à mettre en communication de fluide le premier accès en entrée (51) avec chacun des deux accès en sortie (54 et 56) destinés à communiquer avec chaque sac,
- deux clamps (322, 324) placés respectivement sur chaque portion de la première ligne (57) connectée auxdits deux accès en sortie (54 et 56),

et

- une deuxième ligne (58) destinée à mettre en communication de fluide le deuxième accès en sortie (52) avec chacun des deux accès en entrée (53 et 55) destinés à communiquer avec chaque sac,
- deux autres clamps (321, 323) placés respectivement sur chaque portion de la deuxième ligne (58) connectée auxdits deux accès en entrée (53 et 55).

**17.** Dispositif selon la revendication 15 **caractérisé en ce que** le circuit hydraulique du deuxième organe de réglage (32) comporte :

- une première ligne (57) destinée à mettre en communication de fluide le premier accès en entrée (51) avec chacun des deux accès en sortie (53, 55) destinés à communiquer avec chaque sac et une première vanne trois voies (325) pouvant adopter les deux positions alternées suivantes:

o première position : mise en communication de fluide du premier accès en entrée (51) avec le troisième accès en sortie (53) au niveau du premier sac (11),
o seconde position: mise en communication de fluide du premier accès en entrée (51) avec le cinquième accès en sortie (55) au niveau du deuxième sac (12),

- une deuxième ligne (58) destinée à mettre en communication de fluide le deuxième accès en sortie (52) avec chacun des deux accès en entrée (54, 56) destinés à communiquer avec chaque sac et une deuxième vanne trois voies (326) pouvant adopter les deux positions alternées suivantes correspondantes:

o première position: mise en communication de fluide du deuxième accès en sortie (52) avec le sixième accès en entrée (56), au niveau du deuxième sac (12),
o seconde position: mise en communication de fluide du deuxième accès en sortie (52) avec le quatrième accès en entrée (54), au niveau du premier sac (11).

**18.** Dispositif selon la revendication 16 caractérisé en ce l'unité de contrôle (41) commande simultanément les clamps (321, 323, 323, 324) du deuxième organe de réglage (32) de sorte que les deux phases suivantes sont alternées durant le fonctionnement:

- en première phase: le deuxième sac (12) se charge de liquide tandis que le premier sac (11) se décharge vers l'égout (9),
- en deuxième phase: le premier sac (11) se charge de liquide usé tandis que le deuxième sac (12) se décharge vers l'égout (9),

et en ce que le troisième organe de réglage (33) assure un débit substantiellement continu.

**19.** Dispositif selon une des revendications précédentes **caractérisé en ce que** l'unité de contrôle (41) est capable de calculer la quantité de fluide sortant du filtre (2) et entrant dans la ligne de vidange (8) à partir des signaux reçus du premier moyen de pesage gravimétrique (21) et/ou du second moyen de pesage gravimétrique (22).

**20.** Dispositif selon une des revendications précédentes caractérisé en ce l'unité de contrôle (41) est capable de :

- recevoir l'information de poids du premier moyen de pesage gravimétrique (21) et du second moyen de pesage gravimétrique (22),
- déterminer indépendamment l'état de remplissage de chaque sac,
- commander, à partir de l'état de remplissage de chaque sac, une procédure de charge et décharge alternée et successive des sacs.

**21.** Dispositif selon une des revendications précédentes **caractérisé en ce que** l'unité de contrôle (41) est capable de d'activer une procédure de contrôle comportant les deux phases suivantes alternées:

- dans une première phase : contrôler le débit réel des moyens de réglage (31, 32, 33, 34) comme fonction du profil de débit souhaité et de l'information de poids venant du premier moyen de pesage gravimétrique (21),
- dans une seconde phase : contrôler le débit réel des moyens de réglage (31, 32, 33, 34) comme fonction du profil de débit souhaité et de l'information de poids venant du premier et du second moyens de pesage gravimétrique (21, 22).

**22.** Dispositif selon une des revendications précédentes caractérisé en ce l'unité de contrôle (41) est capable de :

- recevoir l'information de poids du premier moyen de pesage gravimétrique (21) et du second moyen de pesage gravimétrique (22),
- détecter des valeurs de seuil maximal et seuil minimal pour chacun des sacs $P_{1mini}$, $P_{1maxi}$, $P_{2mini}$, $P_{2maxi}$,
- commander à partir de valeurs de seuil une procédure de charge et décharge des sacs selon les étapes suivantes :

    o charge d'un sac et décharge de l'autre sac,
    o détection d'un seuil limite,
    o décharge d'un sac et charge de l'autre sac,
    o détection d'un autre seuil limite.

**Claims**

**1.** Device for treating blood by extracorporeal circulation (1) having:

- a filter (2) having a primary chamber (3) and a secondary chamber (4) separated by a semipermeable membrane (5),
- a blood circuit having an arterial line (6) that is suitable for withdrawing from the patient, the primary chamber (3) of the filter and a venous line (7) that is suitable for returning to the patient,
- a dialysate circuit having the secondary chamber (4) of the filter and a discharging line (8) for the circulation of the used liquid that is suitable for exiting the filter (2) and is suitable for going to a drain (9),
- a first bag (11) in fluid communication with the discharging line (8),
- at least one first gravimetric weighing means (21) associated with the first bag (11),
- fluid flowrate adjusting means (31, 32, 33, 34) that is active on the discharging line (8),
- a control unit (41) connected to the first gravimetric weighing means (21) and to the fluid flowrate adjusting means (31, 32, 33, 34),
- a second bag (12) in fluid communication with the discharging line (8),
- a second gravimetric weighing means (22) associated with the second bag (12) and connected to the control unit (41),

said control unit (41) being able to:

receive the weight signals from the first gravimetric weighing means and command the fluid flowrate adjusting means (31, 32, 33, 34) to load one of the bags (11, 12) with liquid whilst the other bag (12, 11) discharges liquid and vice versa in order to obtain an alternating and subsequent bags loading and discharging procedure and said control unit (41) being able to:

receive the weight information from the first gravimetric weighing means (21) and from the second gravimetric weighing means (22),
calculate the real flowrate of the fluid exiting the filter (2) and compare this real flowrate with a desired fluid flowrate by means of the adjusting means to approach the desired fluid flowrate exiting the filter (2).

**2.** Device according to claim 1, **characterised in that** the control unit (41) is able to calculate, from the weight signals, the quantity of liquid exiting the filter and entering the discharging line (8).

**3.** Device according to claim 1 or 2, **characterised in that** the adjusting means has a first adjusting member (31) that is active upstream of the two bags (11, 12) and **in that** the control unit (41) commands the first adjusting member (31) to ensure the presence of a substantially continuous flowrate during treatment.

**4.** Device according to claim 3, **characterised in that** the adjusting means has a second adjusting member (32) that is active between the two bags (11, 12).

**5.** Device according to claim 4, **characterised in that** the adjusting means has a third adjusting member (33) that is active downstream of the two bags (11, 12).

**6.** Device according to any one of claims 4 to 5, **characterised in that** the discharging line (8) comprises:

- a conduit (80) that is suitable for connecting the filter (2) to the drain (9),
- a first branch (81) connecting the first bag (11) to the conduit (80).
- a second branch (82) connecting the second bag (12) to the conduit (80),
- the second branch (82) being connected to the conduit (80) upstream of the first branch (81),

and **in that** the first adjusting member (31) and the second adjusting member (32) are active on the conduit (80).

7. Device according to claim 6, **characterised in that** each branch (81, 82) has a line with two respective end fittings (811, 812, 821, 822).

8. Device according to claim 6 or 7, **characterised in that** each branch (81, 82) has a direct fitting between the discharging line (80) and a bag opening.

9. Device according to any one of claims 5 and 6 to 8, **characterised in that** the first (31) and third (33) adjusting member are peristaltic pumps and the second adjusting member (32) is a valve.

10. Device according to claim 9, **characterised in that** the first bag (11) is positioned lower than the second bag (12) in such a manner that the first bag (11) is loaded with priority and the second bag (12) discharges into the first bag (11) by gravity when the second adjusting member (32) is open.

11. Device according to claim 9 or 10, **characterised in that** the control unit (41) is able to control the adjusting members (31, 32, 33) according to the following two alternate steps:

    - in the first step: the control unit (41) commands the closure of the second adjusting member (32) for loading the second bag (12) and discharging the first bag (11) into the drain (9),
    - in the second step: the control unit (41) commands the opening of the second adjusting member (32) and the arrest of the third adjusting member (33) for the discharge of the second bag (12) and the loading of the first bag (11).

12. Device according to claims 5 and 7, **characterised in that** the adjusting means has a fourth adjusting member (34) that is active on the first branch (81) between the fitting (811) and the first bag (11).

13. Device according to claim 12, **characterised in that** the first and the fourth adjusting members (31, 34) are peristaltic pumps and the second and third adjusting members (32, 33) are valves.

14. Device according to claim 13, **characterised in that** the control unit (41) is able to control the flowrate adjusting means (31, 32, 33, 34) according to the following two alternate steps:

    - in the first step: the control unit (41) commands the closure of the second adjusting member (32), the opening of the third adjusting member (33), the driving of the fourth adjusting member (34) in the bag-conduit direction,
    - in the second step: the control unit (41) commands the opening of the second adjusting member (32), the closure of the third adjusting member (33), the driving of the fourth adjusting member (34) in the conduit-bag direction.

15. Device according to any one of claims 4 to 5, **characterised in that** the second adjusting member (32) has a hydraulic circuit having six accesses (51, 52, 53, 54, 55, 56) distributed in the following manner:

    a, a first inlet access (51) suitable for being in fluid communication with the inlet portion of the discharging line (8) that is suitable for being connected to the filter (2),
    b. a second outlet access (52) suitable for being in fluid communication with the outlet portion of the discharging line (8) that is suitable for being connected to the drain (9),
    c. a third inlet access (53) and a fourth outlet access (54) that are each suitable for being in fluid communication with the first bag (11),
    d. a fifth inlet access (55) and a sixth outlet access (56) that are each suitable for being in fluid communication with the second bag (12).

16. Device according to claim 15, **characterised in that** the hydraulic circuit of the second adjusting member (32) has:

- a first line (57) suitable for placing the first inlet access (51) in fluid communication with each of the two outlet accesses (54 and 56) that are suitable for communicating with each bag;
- two clamps (322, 324) placed respectively on each portion of the first line (57) that is connected to said two outlet accesses (54 and 56),
and
- a second line (58) suitable for placing the second outlet access (52) in fluid communication with each of the two inlet accesses (53 and 55) suitable for communicating with each bag,
- two other clamps (321, 323) placed respectively on each portion of the second line (58) connected to said two inlet accesses (53 and 55).

17. Device according to claim 15, **characterised in that** the hydraulic circuit of the second adjusting member (32) has:

- a first line (57) suitable for placing the first inlet access (51) in fluid communication with each of the two outlet accesses (53, 55) suitable for communicating with each bag and a first three-way valve (325) that is able to adopt the two following alternate positions:
- first position: placing the first inlet access (51) in fluid communication with the third outlet access (53) at the level of the first bag (11),
- second position: placing the first inlet access (51) in fluid communication with the fifth outlet access (55) at the level of the second bag (12),
- a second line (58) suitable for placing the second outlet access (52) in fluid communication with each of the two inlet accesses (54, 56) suitable for communicating with each bag and a second three-way valve (326) that is able to adopt the following two alternate corresponding positions:
- first position: placing the second outlet access (52) in fluid communication with the sixth inlet access (56) at the level of the second bag (12),
- second position: placing the second outlet access (52) in fluid communication with the fourth inlet access (54) at the level of the first bag (11).

18. Device according to claim 16, **characterised in that** the control unit (41) commands simultaneously the clamps (321, 323, 323, 324) of the second adjusting member (32) in such a manner that the two following steps alternate during operation:

- in the first step: the second bag (12) is loaded with liquid whilst the first bag (11) discharges towards the drain (9),
- in the second step: the first bag (11) is loaded with used liquid whilst the second bag (12) discharges towards the drain (9),
and **in that** the third adjusting member (33) ensures a substantially continuous flowrate.

19. Device according to any preceding claim, **characterised in that** the control unit (41) is able to calculate the quantity of fluid exiting the filter (2) and entering the discharging line (8) from the signals received from the first gravimetric weighing means (21) and/or from the second gravimetric weighing means (22).

20. Device according to any preceding claim, **characterised in that** the control unit (41) is able to:

- receive the weight information from the first gravimetric weighing means (21) and from the second gravimetric weighing means (22),
- determine independently the filling status of each bag,
- command, from the filling status of each bag, an alternating and subsequent bags loading and discharging procedure.

21. Device according to any preceding claim, **characterised in that** the control unit (41) is able to activate a control procedure having the following two alternate steps:

- in a first step: controlling the real flowrate of the adjusting means (31, 32, 33, 34) as a function of the desired flowrate profile and of the weight information coming from the first gravimetric weighing means (21),
- in a second step: checking the real flowrate of the adjusting means (31, 32, 33, 34) as a function of the designed flowrate profile and of the weight information coming from the first and from the second gravimetric weighing means (21, 22).

22. Device according to any preceding claim, **characterised in that** the control unit (41) is able to:

- receive the weight information of the first gravimetric weighing means (21) and of the second gravimetric weighing means (22),
- detect the maximum and minimum threshold values for each of the bags $P_{1mini}$, $P_{1maxi}$, $P_{2mini}$, $P_{2maxi}$,
- command, from threshold values, a bags loading and discharging procedure according to the following steps:
- loading one bag and discharging the other bag,
- detecting a limit threshold,
- discharging one bag and loading the other,
- detecting another limit threshold.

**Patentansprüche**

1. Vorrichtung zur Blutbehandlung durch einen extrakorporalen Kreislauf (1), beinhaltend:

 - einen Filter (2), aufweisend eine Hauptkammer (3) und eine Nebenkammer (4), die durch eine semipermeable Membran (5) getrennt sind,
 - einen Blutkreislauf, beinhaltend eine Arterienleitung (6), die dazu bestimmt ist, aus dem Patienten herauszutreten, die Hauptkammer (3) des Filters und eine Venenleitung (7), die dazu bestimmt ist, in den Patienten zurückzukehren,
 - einen Dialysatkreislauf, beinhaltend die Nebenkammer (4) des Filters und eine Ablassleitung (8) für den Kreislauf der gebrauchten Flüssigkeit, die dazu bestimmt ist, aus dem Filter (2) herauszutreten und dazu bestimmt ist, zu einem Abfluss (9) zu laufen,
 - einen ersten Beutel (11), der mit der Ablassleitung (8) in Fluidverbindung steht,
 - mindestens ein erstes gravimetrisches Wiegemittel (21), das an den ersten Beutel (11) angeschlossen ist,
 - Mittel zur Regulierung eines Fluiddurchflusses (31, 32, 33, 34), die auf die Ablassleitung (8) einwirken,
 - eine Steuereinheit (41), die mit dem ersten gravimetrischen Wiegemittel (21) und mit den Mitteln zur Regulierung des Fluiddurchflusses (31, 32, 33, 34) verbunden ist,
 - einen zweiten Beutel (12), der mit der Ablassleitung (8) in Fluidverbindung steht,
 - ein zweites gravimetrisches Wiegemittel (22), das an den zweiten Beutel (12) angeschlossen und mit der Steuereinheit (41) verbunden ist,
 wobei die Steuereinheit (41) fähig ist zum:

 Empfangen der Gewichtssignale vom ersten gravimetrischen Wiegemittel und
 Steuern der Mittel zur Regulierung eines Fluiddurchflusses (31, 32, 33, 34) zum Auffüllen eines der Beutel (11, 12) mit Flüssigkeit, während der andere Beutel (12, 11) Flüssigkeit ablässt und umgekehrt, um ein alterniertes und aufeinanderfolgendes Auffüll- und Entleerungsverfahren der Beutel zu erhalten und wobei die Steuereinheit (41) fähig ist zum:
 Empfangen der Gewichtsinformation vom ersten gravimetrischen Wiegemittel (21) und vom zweiten gravimetrischen Wiegemittel (22), Berechnen des realen Durchflusses des vom Filter (2) heraustretenden Fluides und Vergleichen dessen mit einem gewollten Fluiddurchfluss, und Steuern des realen Durchflusses mithilfe der Regulierungsmittel zum Annähern an den gewollten Fluiddurchfluss des vom Filter (2) heraustretenden Fluids.

2. Vorrichtung nach Anspruch 1, **dadurch gekennzeichnet, dass** die Steuereinheit (41) dazu fähig ist, die Menge der aus dem Filter heraustretenden und in die Ablassleitung (8) eintretenden Flüssigkeit aus den empfangenen Gewichtssignalen zu berechnen.

3. Vorrichtung nach Anspruch 1 oder 2, **dadurch gekennzeichnet, dass** die Regulierungsmittel ein erstes Regulierungselement (31) beinhalten, das vor den zwei Beuteln (11, 12) wirkt und dass die Steuereinheit (41) das erste Regulierungselement (31) steuert, um während der Behandlung das Vorhandensein eines im Wesentlichen kontinuierlichen Durchflusses zu garantieren.

4. Vorrichtung nach Anspruch 3, **dadurch gekennzeichnet, dass** die Regulierungsmittel ein zweites Regulierungselement (32) beinhalten, das zwischen den zwei Beuteln (11, 12) wirkt.

5. Vorrichtung nach Anspruch 4, **dadurch gekennzeichnet, dass** die Regulierungsmittel ein drittes Regulierungselement (33) beinhalten, das nach den zwei Beuteln (11, 12) wirkt.

**6.** Vorrichtung nach einem der Ansprüche 4 bis 5, **dadurch gekennzeichnet, dass** die Ablassleitung (8) umfasst:

- einen Schlauch (80), der dazu bestimmt ist, den Filter (2) mit dem Abfluss (9) zu verbinden,
- eine erste Abzweigung (81), die den ersten Beutel (11) mit dem Schlauch (80) verbindet,
- eine zweite Abzweigung (82), die den zweiten Beutel (12) mit dem Schlauch (80) verbindet,
- wobei die zweite Abzweigung (82) vor der ersten Abzweigung (81) an den Schlauch (80) angeschlossen ist,

und dass das erste Regulierungselement (31) und das zweite Regulierungselement (32) auf den Schlauch (80) einwirken.

**7.** Vorrichtung nach Anspruch 6, **dadurch gekennzeichnet, dass** jede Abzweigung (81, 82) eine Leitung mit zwei entsprechenden Endanschlussstücken (811, 812, 821, 822) beinhaltet.

**8.** Vorrichtung nach Anspruch 6 oder 7, **dadurch gekennzeichnet, dass** jede Abzweigung (81, 82) zwischen der Ablassleitung (80) und einer Öffnung eines Beutels ein Direktanschlussstück beinhaltet.

**9.** Vorrichtung nach einem der Ansprüche 5 und 6 bis 8, **dadurch gekennzeichnet, dass** das erste (31) und dritte (33) Regulierungselement Peristaltikpumpen sind und das zweite Regulierungselement (32) ein Ventil ist.

**10.** Vorrichtung nach Anspruch 9, **dadurch gekennzeichnet, dass** der erste Beutel (11) unterhalb des zweiten Beutels (12) liegt, damit der erste Beutel (11) sich vorrangig auffüllen kann und sich der zweite Beutel (12) aufgrund der Schwerkraft in den ersten Beutel (11) entleert, wenn das zweite Regulierungselement (32) geöffnet ist.

**11.** Vorrichtung nach Anspruch 9 oder 10, **dadurch gekennzeichnet, dass** die Steuereinheit (41) dazu fähig ist, die Regulierungselemente (31, 32, 33) nach den zwei folgenden alternierenden Phasen zu steuern:

- in der ersten Phase: die Steuereinheit (41) steuert die Schließung des zweiten Regulierungselements (32) für das Auffüllen des zweiten Beutels (12) und das Entleeren des ersten Beutels (11) in den Abfluss (9),
- in der zweiten Phase: die Steuereinheit (41) steuert die Öffnung des zweiten Regulierungselements (32) und das Anhalten des dritten Regulierungselements (33) für das Entleeren des zweiten Beutels (12) und das Auffüllen des ersten Beutels (11).

**12.** Vorrichtung nach den Ansprüchen 5 und 7, **dadurch gekennzeichnet, dass** die Regulierungsmittel ein viertes Regulierungselement (34) aufweisen, das zwischen dem Anschlussstück (811) und dem ersten Beutel (11) auf die erste Abzweigung (81) einwirkt.

**13.** Vorrichtung nach Anspruch 12, **dadurch gekennzeichnet, dass** das erste und vierte Regulierungselement (31, 34) Peristaltikpumpen und das zweite und dritte Regulierungselement (32, 33) Ventile sind.

**14.** Vorrichtung nach Anspruch 13, **dadurch gekennzeichnet, dass** die Steuereinheit (41) dazu fähig ist, die Elemente (31, 32, 33, 34) zur Durchflussregulierung nach den zwei folgenden alternierenden Phasen zu steuern:

- in der ersten Phase: die Steuereinheit (41) steuert die Schließung des zweiten Regulierungselements (32), die Öffnung des dritten Regulierungselements (33), die Betätigung des vierten Regulierungselements (34) in der Richtung Beutel-Schlauch,
- in der zweiten Phase: die Steuereinheit (41) steuert die Öffnung des zweiten Regulierungselements (32), die Schließung des dritten Regulierungselements (33), die Betätigung des vierten Regulierungselements (34) in der Richtung Schlauch-Beutel.

**15.** Vorrichtung nach einem der Ansprüche 4 bis 5, **dadurch gekennzeichnet, dass** das zweite Regulierungselement (32) einen Hydraulikkreis beinhaltet, der sechs Zugänge (51, 52, 53, 54, 55, 56) aufweist, die wie folgt unterteilt sind:

a. ein erster eingehender Zugang (51), der dazu bestimmt ist, mit dem Eingangsteil der Ablassleitung (8), die dazu bestimmt ist, mit dem Filter (2) verbunden zu sein, in Fluidverbindung zu stehen,
b. ein zweiter abgehender Zugang (52), der dazu bestimmt ist, mit dem Ausgangsteil der Ablassleitung (8), die dazu bestimmt ist, mit dem Abfluss (9) verbunden zu sein, in Fluidverbindung zu stehen,
c. ein dritter eingehender Zugang (53) und ein vierter abgehender Zugang (54), die jeweils dazu bestimmt sind, mit dem ersten Beutel (11) in Fluidverbindung zu stehen,

d. ein fünfter eingehender Zugang (55) und ein sechster abgehender Zugang (56), die jeweils dazu bestimmt sind, mit dem zweiten Beutel (12) in Fluidverbindung zu stehen.

16. Vorrichtung nach Anspruch 15, **dadurch gekennzeichnet, dass** der Hydraulikkreis des zweiten Regulierungselements (32) Folgendes beinhaltet:

- eine erste Leitung (57), die dazu bestimmt ist, den ersten eingehenden Zugang (51) mit jedem der zwei abgehenden Zugänge (54 und 56), die dazu bestimmt sind, mit jedem Beutel verbunden zu sein, in Fluidverbindung zu setzen,
- zwei Klemmen (322, 324) die jeweils auf jedem Teil der ersten Leitung (57), die mit den beiden abgehenden Zugängen (54 und 56) verbunden ist, liegen
und
- eine zweite Leitung (58), die dazu bestimmt ist, den zweiten abgehenden Zugang (52) mit jedem der zwei eingehenden Zugänge (53 und 55), die dazu bestimmt sind, mit jedem Beutel verbunden zu sein, in Fluidverbindung zu setzen,
- zwei weitere Klemmen (321, 323), die jeweils auf jedem Teil der zweiten Leitung (58), die mit den zwei eingehenden Zugängen (53 und 55) verbunden ist, liegen.

17. Vorrichtung nach Anspruch 15, **dadurch gekennzeichnet, dass** der Hydraulikkreis des zweiten Regulierungselements (32) beinhaltet:

- eine erste Leitung (57), die dazu bestimmt ist, den ersten eingehenden Zugang (51) mit jedem der zwei abgehenden Zugänge (53 und 55), die dazu bestimmt sind, mit jedem Beutel verbunden zu sein, in Fluidverbindung zu setzen und ein erstes Dreiwegeventil (325), das die zwei folgenden alternierenden Positionen annehmen kann:
- erste Position: Setzen des ersten eingehenden Zugangs (51) in Fluidverbindung mit dem dritten abgehenden Zugang (53) in Höhe des ersten Beutels (11),
- zweite Position: Setzen des ersten eingehenden Zugangs (51) in Fluidverbindung mit dem fünften abgehenden Zugang (55) in Höhe des zweiten Beutels (12),
- eine zweite Leitung (58), die dazu bestimmt ist, den zweiten abgehenden Zugang (52) mit jedem der zwei eingehenden Zugänge (54 und 56), die dazu bestimmt sind, mit jedem Beutel verbunden zu sein, in Fluidverbindung zu setzen und ein zweites Dreiwegeventil (326), das die zwei folgenden entsprechenden alternierenden Positionen annehmen kann:
- erste Position: Setzen des zweiten abgehenden Zugangs (52) in Fluidverbindung mit dem sechsten eingehenden Zugang (53) in Höhe des zweiten Beutels (12),
- zweite Position: Setzen des zweiten abgehenden Zugangs (52) in Fluidverbindung mit dem vierten eingehenden Zugang (54) in Höhe des ersten Beutels (11).

18. Vorrichtung nach Anspruch 16, **dadurch gekennzeichnet, dass** die Steuereinheit (41) gleichzeitig die Klemmen (321, 323, 323, 324) des zweiten Regulierungselements (32) steuert, damit die zwei folgenden Phasen während des Betriebs alternierend sind:

- in der ersten Phase: der zweite Beutel (12) füllt sich mit Flüssigkeit auf, während der erste Beutel (11) Flüssigkeit in Richtung Abfluss (9) ablässt,
- in der zweiten Phase: der erste Beutel (11) füllt sich mit gebrauchten Flüssigkeit auf, während sich der zweite Beutel (12) Flüssigkeit in Richtung Abfluss (9) ablässt

und dass das dritte Regulierungselement (33) einen im Wesentlichen kontinuierlichen Durchfluss gewährleistet.

19. Vorrichtung nach einem der vorangehenden Ansprüche, **dadurch gekennzeichnet, dass** die Steuereinheit (41) dazu fähig ist, die Menge des aus dem Filter (2) heraustretenden und in die Ablassleitung (8) eintretenden Fluids bei den vom ersten gravimetrischen Wiegemittel (21) und/oder vom zweiten gravimetrischen Wiegemittel (22) empfangenen Gewichtssignalen zu berechnen.

20. Vorrichtung nach einem der vorangehenden Ansprüche, **dadurch gekennzeichnet, dass** die Steuereinheit (41) fähig ist zum:

- Empfangen der Gewichtsinformation vom ersten gravimetrischen Wiegemittel (21) und vom zweiten gravimetrischen Wiegemittel (22),

- unabhängiges Bestimmen des Füllzustandes eines jeden Beutels,
- ab dem Füllzustand eines jeden Beutels Steuern eines alternierten und aufeinanderfolgenden Auffüll- und Entleerungsverfahrens der Beutel.

21. Vorrichtung nach einem der vorangehenden Ansprüche, **dadurch gekennzeichnet, dass** die Steuereinheit (41) dazu fähig ist, ein Steuerverfahren zu aktivieren, das die zwei folgenden alternierten Phasen beinhaltet:

- in einer ersten Phase: Steuern des realen Durchflusses der Regulierungsmittel (31, 32, 33, 34) in Abhängigkeit vom Profil des gewollten Durchflusses und der Gewichtsinformation, die vom ersten gravimetrischen Wiegemittel (21) kommt,
- in einer zweiten Phase: Steuern des realen Durchflusses der Regulierungsmittel (31, 32, 33, 34) in Abhängigkeit vom Profil des gewollten Durchflusses und der Gewichtsinformation, die vom ersten und vom zweiten gravimetrischen Wiegemittel (21, 22) kommt.

22. Vorrichtung nach einem der vorangehenden Ansprüche, **dadurch gekennzeichnet, dass** die Steuereinheit (41) fähig ist zum:

- Empfangen der Gewichtsinformation des ersten gravimetrischen Wiegemittels (21) und des zweiten gravimetrischen Wiegemittels (22),
- Ermitteln von maximalen und minimalen Schwellenwerten für jeden der Beutel $P_{1mini}$, $P_{1maxi}$, $P_{2mini}$, $P_{2maxi}$,
- ab den Schwellenwerten Steuern eines Auffüll- und Entleerungsverfahrens der Beutel nach den folgenden Phasen:
- Auffüllen eines Beutels und Entleeren des anderen Beutels,
- Ermitteln eines Grenzwertes,
- Entleeren eines Beutels und Auffüllen des anderen Beutels,
- Ermitteln eines anderen Grenzwertes.

Etat de l'art

Fig. 1

1er Mode de réalisation

(1)

Fig. 2

phase 1

fermé

Fig. 3

phase 2

ouvert

Fig. 4

EP 1 635 895 B1

Fig. 5

2eme Mode de réalisation

(1)

CPU — 41

P.maxi.2 — 22
P.mini.2 — 12

82

liquide de dialyse

(2)

P.maxi.1
P.mini.1 — 11

34b
81
34
33 33b 811 32 32b 80

P.maxi.1
P.mini.1

31b
812
9
31 liquide usé

5
4 3
6
7

Fig. 6

EP 1 635 895 B1

phase 1

ouvert       fermé

Fig. 7

phase 2

fermé       ouvert

Fig. 8

3 ième Mode de réalisation

(1)

Fig. 9

phase 1

fermé

ouvert ouvert ▷ ◁

ouvert ▷ ◁ fermé

Fig. 10

phase 2

ouvert ▷ ◁ fermé

fermé ouvert ▷ ◁

Fig. 11

EP 1 635 895 B1

4 ième Mode de réalisation

Fig. 12

Fig. 13

phase 1

Fig. 14

phase 2

## RÉFÉRENCES CITÉES DANS LA DESCRIPTION

**Documents brevets cités dans la description**

- EP 0796997 A
- US 4859319 A
- US 50430745 B